(19) Europäisches Patentamt
European Patent Office
Office européen des brevets

(11) EP 1 333 019 A1

(12) **EUROPEAN PATENT APPLICATION**

(43) Date of publication:
**06.08.2003 Bulletin 2003/32**

(51) Int Cl.[7]: **C07C 29/143**, C07C 35/14

(21) Application number: **03002527.4**

(22) Date of filing: **05.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **05.02.2002 JP 2002028217**

(71) Applicant: **MITSUBISHI CHEMICAL CORPORATION**
**Chiyoda-ku, Tokyo 100-0005 (JP)**

(72) Inventors:
- **Ichikawa, Shuji**
  **Inashiki-gun, Ibaraki 300-0332 (JP)**
- **Urata, Hisao**
  **Inashiki-gun, Ibaraki 300-0332 (JP)**
- **Suzuki, Shihomi**
  **Inashiki-gun, Ibaraki 300-0332 (JP)**

(74) Representative: **VOSSIUS & PARTNER**
**Siebertstrasse 4**
**81675 München (DE)**

(54) **Method for producing 1,3-cyclohexanediol compound**

(57) A method for producing 1,3-cyclohexanediol compounds, particularly a 1,3-cyclohexanediol compound rich in cis-form. A 1,3-cyclohexanediol compound is reduced with a boron hydride compound such as sodium borohydride. Selectivity of cis-form is improved when an alkali metal compound and/or an alkaline earth metal compound, particularly a halide, is allowed to exist in the reaction system.

EP 1 333 019 A1

## Description

This invention relates to a method for producing 1,3-cyclohexanediol compounds from 1,3-cyclohexanedione compounds. 1,3-Cyclohexanediol compounds are useful as, e.g., starting materials for preparing medicaments, agricultural chemicals and fine chemicals.

In addition, according to the invention, 1,3-cyclohexanediol compounds rich in cis-form can also be produced.

As the method for producing 1,3-cyclohexanediol, a method in which resorcinol is hydrogenated at a temperature of from 90 to 95°C in the presence of Raney nickel is conventionally known (e.g., *Journal of Organic Chemistry*, Vol. 26, p. 1625 (1961)). However, this method requires a large amount of Raney nickel, namely 50% by weight or more based on the substrate resorcinol, is apt to undergo influence of reaction conditions such as agitation speed, and has a problem regarding reproducibility of the reaction. In addition, since equipment such as a high pressure instrument is required for the reduction reaction, this is not advantageous as an industrial production method.

Also known are a method in which cyclohexane is used as the starting material which is oxidized with oxygen in the presence of ruthenium (III) ethylenediaminetetraacetate and ascorbic acid (*Recent Advances in Basic and Applied Aspects of Industrial Catalysis*, Vol. 113, p. 897 (1998)) and a method in which cyclohexanol is used as the starting material which is oxidized with perchloric acid and a hydrogen peroxide aqueous solution in the presence of iron (II) perchlorate (*Journal of The American Chemical Society,* Vol. 96, p. 5274 (1974)). However, each of these production methods of 1,3-cyclohexanediol making use of oxidation reaction is not desirable as an industrial production method because of the low yield.

In addition, when it is particularly desired to produce a cis-form 1,3-cyclohexanediol selectively, it cannot be produced by these methods. Only available methods are techniques for obtaining cis-1,3-cyclohexanediol by firstly converting its cis-form and trans-form as a mixture into derivatives and then separating the isomers, such as a method in which cis-1,3-cyclohexanediol is produced by converting a mixture of cis-and trans-1,3-cyclohexanediol into dibenzoyl derivatives, separating the cis-form and trans-form derivatives making use of a silica gel chromatography and then eliminating the benzyl group using sodium methoxide (cf. Reference Examples 4-1 and 4-2 in JP-A-10-45793) and a method in which cis-1,3-cyclohexanediol is produced by converting a mixture of cis- and trans-1,3-cyclohexanediol into cyclic boric acid esters using trihexylboric acid ester, separating the esters by distillation making use of the difference in boiling point between the cis-form and trans-form, and then eliminating the boric acid ester (*Journal of The Chemical Society, p.* 922 (1961)). However, these methods cannot be said as practical production methods, because they require many steps of converting a cis- and trans-form mixture into derivatives, separating them and then converting them into the cis-1,3-cyclohexanediol of interest and because of the low total yield

Under such circumstances, the present invention contemplates providing a method for industrially and conveniently producing 1,3-cyclohexanediol compounds, particularly a method for producing 1,3-cyclohexanediol compounds rich in cis-form, industrially advantageously and efficiently by a relatively simple process with a low cost.

As a solution according to the present invention it has been found that a 1,3-cyclohexanediol compound represented by the following general formula (2) can be efficiently produced by reducing a 1,3-cyclohexanedione compound represented by the following general formula (1) with a boron hydride compound, and that selectivity of its cis-form can be improved by carrying out the reaction using a specified reagent.

Accordingly, the gist of the invention resides in a method for producing a 1,3-cyclohexanediol compound represented by the following general formula (2)

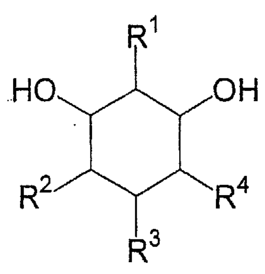

(2)

(wherein $R^1$, $R^2$, $R^3$ and $R^4$ each independently represents hydrogen atom, a halogen atom, an alkoxycarbonyl group, an alkyl group which may have one or more substituent(s), an alkoxy group which may have one or more substituent (s) or phenyl group which may have one or more substituent(s)) which comprises reducing a 1,3-cyclohexanedione compound represented by the following general formula (1)

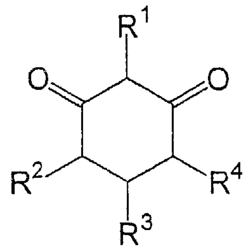

(1)

(wherein $R^1$, $R^2$, $R^3$ and $R^4$ are as defined in the formula (2)) with a boron hydride compound.

The following describes the invention in detail.

(Starting Material)

The 1,3-cyclohexanedione compound to be used in the present invention as the starting material is represented by the general formula (1). This compound can be optionally produced by known methods such as a method in which so-called proton-transferring reduction is carried out by reducing a 1,3-dihydroxybenzene compound represented by the following general formula (3) using a hydrogen donor such as sodium formate in the presence of a noble metal catalyst, and then the product is neutralized with an acid (cf. JP-A-10-87548).

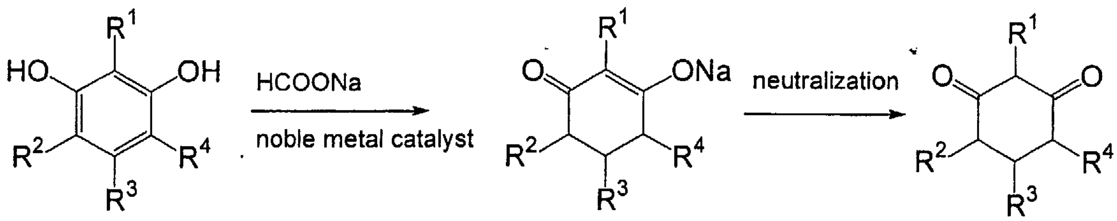


(3)

(1)

(In the formula, $R^1$, $R^2$, $R^3$ and $R^4$ are as defined in the foregoing.)

Each of $R^1$, $R^2$, $R^3$ and $R^4$ independently represents hydrogen atom; a halogen atom such as chlorine atom, bromine atom or iodine atom; an alkoxycarbonyl group; an alkyl group which may have one or more substituent(s); an alkoxy group which may have one or more substituent(s); or phenyl group which may have one or more substituent(s).

Examples of the alkoxycarbonyl group include straight, branched or cyclic alkoxycarbonyl groups such as methoxycarbonyl group, ethoxycarbonyl group, n-propoxycarbonyl group, i-propoxycarbonyl group, n-butoxycarbonyl group, i-butoxycarbonyl group, t-butoxycarbonyl group, n-pentyloxycarbonyl group, n-hexyloxycarbonyl group, cyclopropyloxycarbonyl group, cyclobutyloxycarbonyl group, cyclopentyloxycarbonyl group, and cyclohexyloxycarbonyl group, of which straight or branched alkoxycarbonyl groups are preferred and those having 7 or less carbon atoms are further preferred.

Examples of the alkyl group include straight, branched or cyclic alkyl groups such as methyl group, ethyl group, n-propyl group, i-propyl group, n-butyl group, i-butyl group, t-butyl group, n-pentyl group, n-hexyl group, cyclopropyl group, cyclobutyl group, cyclopentyl group and cyclohexyl group, of which those having 6 or less carbon atoms are preferred.

Examples of the alkoxy group include straight, branched or cyclic alkyl groups such as methoxy group, ethoxy group, n-propoxy group, i-propoxy group, n-butoxy group, i-butoxy group, t-butoxy group, n-pentyloxy group, n-hexyloxy group, cyclopropyloxy group, cyclobutyloxy group, cyclopentyloxy group and cyclohexyloxy group, of which straight or branched alkoxy groups are preferred and those having 6 or less carbon atoms are further preferred.

The substituent of the alkyl group, alkoxy group and phenyl group is not particularly limited with the proviso that it is a group inert to the reduction reaction of ketone, and its illustrative examples include the above-described halogen atoms, alkyl groups and alkoxy groups; aryl groups such as phenyl group and naphthyl group; and alkoxycarbonylalkyl groups wherein the alkoxycarbonyl group is preferably a group represented by the following general formula (4)

$$-(CH_2)_nCOOR^5 \quad (4)$$

(in the formula, n is an integer of from 0 to 6, and $R^5$ represents hydrogen atom or a $C_1$-$C_6$ alkyl group which may have one or more substituent(s)); of which a halogen atom, a $C_1$-$C_4$ alkyl group, a $C_1$-$C_4$ alkoxy group or phenyl group is preferred. Also, these may be further substituted with the same groups already described.

As the $R^1$, $R^2$, $R^3$ and $R^4$, hydrogen atom, a $C_2$-$C_4$ alkoxycarbonyl group, a $C_1$-$C_4$ alkyl group, a $C_1$-$C_4$ haloalkyl group or benzyl group is desirable, and hydrogen atom or a $C_1$-$C_4$ alkyl group is more desirable.

Examples of the 1,3-cyclohexanedione compound represented by the general formula (1) include 1,3-cyclohexanedione, 2-methyl-1,3-cyclohexanedione, 5-methyl-1,3-cyclohexanedione, 5-ethyl-1,3-cyclohexanedione, 5-n-propyl-1,3-cyclohexanedione, 5-i-propyl-1,3-cyclohexanedione, 5-i-butyl-1,3-cyclohexanedione, 2,5-dimethyl-1,3-cyclohexanedione, 4,6-dimethyl-1,3-cyclohexanedione, 2,4,6-trimethyl-1,3-cyclohexanedione, 1-methoxycarbonyl-2,4-dioxo-6-methylcyclohexane and 1-methoxycarbonyl-2,4-dioxo-6-phenylcyclohexane.

(Boron hydride compound)

According to the production method of the present invention, a boron hydride compound is used as a reducing agent.

As the boron hydride compound, boron hydride compounds of the group I, group II, group XII or group XIII elements of the periodic table, such as lithium borohydride, sodium borohydride, potassium borohydride, beryllium borohydride, magnesium borohydride, calcium borohydride, barium borohydride, zinc borohydride and aluminum borohydride can be generally exemplified, of which sodium borohydride or zinc borohydride is preferable mainly from the economical point of view, and sodium borohydride is particularly preferable.

As these boron hydride compounds, commercially available products may be used as such or they may be prepared optionally by known methods. For example, sodium borohydride can be prepared from a boric acid ester and sodium hydride, and other borohydride compounds can also be prepared by reactions of sodium borohydride with corresponding metal halides, such as a reaction of sodium borohydride with calcium chloride in the case of calcium borohydride.

When a boron hydride compound is used by preparing it, it may be prepared in a reaction vessel and used as such in the reduction reaction.

A reaction formula of the reduction of a 1,3-cyclohexanedione compound into a 1,3-cyclohexanediol compound using a boron hydride compound as the reducing agent is shown below as a typical example when sodium borohydride is used as the reducing agent.

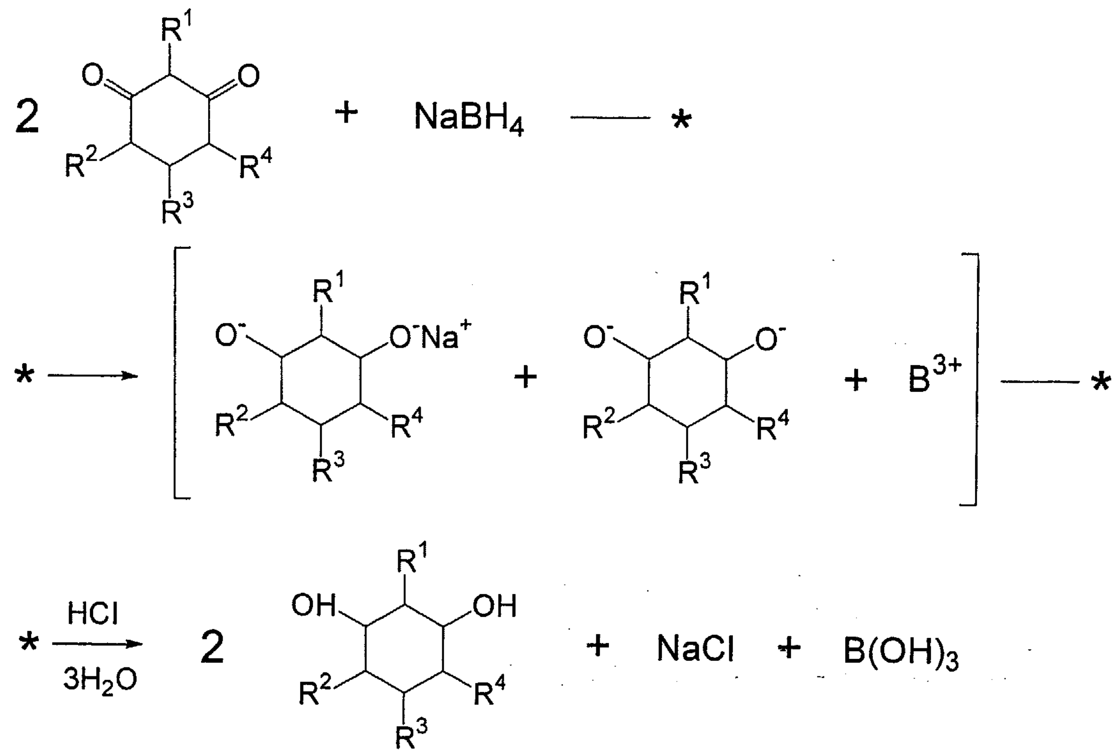


The boron hydride compound is generally used in an amount of the theoretically equivalent (2 moles) or more as hydride atom based on the starting material 1,3-cyclohexanedione compound, but in reality, it is desirable to use it in a slightly excess amount or more than the theoretical equivalent, and illustratively, it is used in an amount of 2.4 moles or more, more preferably 3 moles or more and further preferably 4 moles or more. However, too excess amount will not bear proportionally increased effects but rather cause problems such as complex after-treatment, so that it is used within the range of generally 80 moles or less, preferably 40 moles or less, more preferably 16 moles or less, and further preferably 8 moles or less.

According to the method of the present invention, a cis-form 1,3-cyclohexanediol compound is obtained by carrying out the reaction in the presence of one or two or more metal compounds selected from the group I, group II, group XII or group XIII elements of the periodic table, in addition to the boron hydride compound. In that case, it is desirable that kinds of the metal element to be used as the boron hydride compound are different from the kinds of metal element to be used in other metal compound. Particularly, as the metal compound to be coexisted with the boron hydride compound, the use of a metal compound selected from the group I or group II elements of the periodic table further improves the cis-form selectivity and therefore is desirable.

It is desirable that the metal compounds selected from the group I, group II, group XII or group XIII elements of the periodic table are soluble in solvent.

As the compounds selected from the group I of the periodic table, hydroxides, inorganic acid salts, organic acid salts and alkoxides of alkali metals can be exemplified. For example, nitrates, halides, carbonates, bicarbonates, sulfates, phosphates, borates, chromates and molybdates can be exemplified as the inorganic acid salts, and carboxylic acid salts such as oxalates and acetates can be exemplified as the organic acid salts. Their illustrative examples include cesium nitrate, cesium hydroxide, cesium chloride, cesium bromide, cesium fluoride, cesium iodide, cesium carbonate, lithium nitrate, lithium hydroxide, lithium chloride, lithium bromide, lithium fluoride, lithium iodide, lithium carbonate, lithium oxalate, lithium sulfate, lithium borate, lithium chromate, lithium molybdate, sodium nitrate, sodium carbonate, sodium bicarbonate, sodium acetate, sodium borate, sodium chromate, sodium ethoxide, sodium bromide, sodium fluoride, sodium iodide, potassium nitrate and rubidium nitrate. Among these compounds, halides, borates and sulfates are desirable because they show good cis-form selectivity. Most preferred are halides, particularly lithium halides. Among the lithium halides, lithium chloride or lithium bromide provides high cis-form selectivity.

Also, as the compounds selected from the group II of the periodic table, hydroxides, inorganic acid salts, organic acid salts and alkoxides of alkaline earth metals can be exemplified. For example, nitrates, halides, carbonates, bicarbonates, sulfates, phosphates, borates, chromates and molybdates can be exemplified as the inorganic acid salts, and carboxylic acid salts such as oxalates and acetates can be exemplified as the organic acid salts. Their illustrative examples include beryllium nitrate, magnesium nitrate, magnesium carbonate, magnesium sulfate, magnesium

oxalate, magnesium ethoxide, magnesium chloride, magnesium bromide, magnesium fluoride, magnesium iodide, calcium nitrate, calcium hydroxide, calcium chloride, calcium bromide, calcium fluoride, calcium iodide, calcium acetate, calcium sulfate, calcium molybdate, barium nitrate, barium hydroxide, barium chloride, barium sulfate, strontium nitrate, strontium hydroxide and strontium chloride. Among these compounds, halides, borates and sulfates are preferable. Further preferred are magnesium chloride, magnesium bromide and magnesium sulfate, and magnesium chloride and magnesium bromide are particularly preferred because they provide high cis-form selectivity.

Also, as the compounds selected from the group XII and group XIII of the periodic table, hydroxides, inorganic acid salts, organic acid salts and alkoxides of zinc and aluminum can be exemplified. For example, nitrates, halides, carbonates, sulfates, phosphates, borates and chromates can be exemplified as the inorganic acid salts, and carboxylic acid salts such as oxalates and acetates can be exemplified as the organic acid salts. Their illustrative examples include zinc nitrate, aluminum nitrate, zinc chloride, aluminum chloride, zinc bromide, aluminum bromide, zinc iodide, aluminum iodide, zinc carbonate, zinc sulfate, aluminum sulfate, zinc phosphate, aluminum phosphate, zinc borate, zinc chromate, zinc oxalate, aluminum oxalate, zinc acetate and aluminum acetate. Among these compounds, halides are preferred.

Using amount of these metal compounds selected from the group I, group II, group XII or group XIII elements of the periodic table is not particularly limited, but it is generally within the range of from 0.3 to 10 moles, preferably within the range of from 0.5 to 5 moles, based on the starting material 1,3-cyclohexanedione compound. Their use within the range of from 0.5 to 2 moles is particularly desirable in view of the economical point. In addition, using ratio of these metal compounds selected from the group I, group II, group XII or group XIII elements of the periodic table to the boron hydride compound is not particularly limited too, but it is within the range of generally from 0.1 to 10 moles, preferably within the range of from 0.2 to 5 moles, particularly within the range of from 0.5 to 2 moles, in view of the economical point.

If desired, two or more of these metal compounds selected from the group I, group II, group XII or group XIII elements of the periodic table may be jointly used with no problems.

(Solvent)

Any solvent can be used in the reaction of the invention, with the proviso that it can dissolve the starting material 1,3-cyclohexanedione compound and does not hinder the reduction reaction, and its examples include alcohols such as methanol, ethanol, propanol, butanol, phenol and benzyl alcohol; ethers such as diethyl ether, tetrahydrofuran and dioxane; and aromatic hydrocarbons such as benzene, toluene and xylene. These solvents may be optionally mixed.

Among these solvents, ethers or aromatic hydrocarbons are desirable.

(Reaction conditions)

The reaction may be carried out in the air or in an atmosphere of inert gas such as nitrogen or argon.

Reaction mode of this reaction is not particularly limited, but preferred is a mode in which a predetermined amount of a boron hydride compound is dissolved in a solvent in advance, together with a metal compound selected from the group I, group II, group XII or group XIII elements of the periodic table as occasion demands, and subjected to aging for a predetermined period of time, and then the reaction is carried out by adding the starting material 1,3-cyclohexanedione compound.

The aging temperature is within the range of generally 0°C or more, preferably 10°C or more, and optionally set within the range of generally reflux temperature or less of the solvent used. The aging period has a tendency to be shortened when the aging temperature is high, and in the case of around room temperature for example, it is desirable to carry out the aging for 6 hours or more, preferably 12 hours or more, but when the aging temperature is about 60°C, aging becomes sufficient within 12 hours, e.g., about several hours.

The reaction temperature after the addition of the 1,3-cyclohexanedione compound can be optionally set within the range of from generally 10°C or more, preferably 20°C or more, to reflux temperature of the solvent or less, preferably 80°C or less. Alternatively, the reaction may be carried out directly at the aging temperature.

Though the reaction time varies depending on the reaction scale and reaction temperature, it is optionally set within the range of from generally 0.5 hour or more, preferably several hours (e.g., 2 to 3 hours) or more, to 24 hours or less, preferably 12 hours or less. Though the reaction progresses at a low temperature, it may be carried out with heating in order to improve the reaction rate. The reaction is carried out at a temperature of generally from 0 to 200°C, preferably from 20 to 150°C.

After carrying out the reaction for a predetermined period of time, unreacted boron hydride compound is decomposed by adding aqueous solution of a mineral acid such as hydrochloric acid, sulfuric acid or nitric acid to the reaction solution, the solvent is evaporated as occasion demands and then the 1,3-cyclohexanediol compound of

interest is isolated by general techniques such as extraction, distillation and crystallization.

Next, the invention is described further illustratively based on examples. In this connection, yield, conversion ratio and stereoselectivity were calculated as follows.

The yield and stereoselectivity were calculated based on the following formulae, by determining formed product in the reaction solution by a gas chromatography using normal nonane as the internal standard.

$$\text{Yield (\%)} = \frac{\text{mol of intended product}}{\text{mol of charged starting material}} \times 100$$

$$\text{Stereoselectivity (\%)} = \frac{\text{mol of cis- or trans-1,3-cyclohexanediol compound}}{\text{total mol of 1,3-cyclohexanediol compound}} \times 100$$

Also, the conversion ratio was calculated based on the following formula, by determining unreacted starting material in the reaction solution by a gas chromatography using anisole as the internal standard.

$$\text{Conversion ratio (\%)} = \frac{\text{mol of charged starting material - mol of unreacted starting material}}{\text{mol of charged starting material}} \times 100$$

Example 1

A four neck flask equipped with a condenser, a thermometer and a mechanical stirrer was charged with 5.08 g (134.3 mmol) of sodium borohydride, 50 ml of tetrahydrofuran and 492.9 mg (3.84 mmol) of normal nonane as the internal standard for analysis. After further adding 6.15 g (64.6 mmol) of magnesium chloride, the contents were heated to 60°C and stirred for 1 hour by keeping this temperature. Thereafter, the temperature was decreased to 25°C and the stirring was continued for 2 hours. A 7.54 g (67.2 mmol) portion of 1,3-cyclohexanedione dissolved in 100 ml of tetrahydrofuran was added dropwise to this mixed solution spending 30 minutes. After completion of the dropwise addition, the mixture was heated to 60°C and allowed to undergo the reaction for 3 hours by keeping this temperature. After spontaneous cooling of the reaction solution and subsequent cooling to about 0°C, 23 ml of water was added thereto spending 80 minutes. Thereafter, this was returned to room temperature and stirred for 1 hour. The thus obtained solution was quantitatively analyzed by a gas chromatography and a liquid chromatography to find that 1,3-cyclohexanediol was formed with a yield of 95.7%. The by-products were cyclohexanol (0.1%) and 2-cyclohexen-1-ol (1.3%). Also, conversion ratio of 1,3-cyclohexanedione was 97.3%, and selectivity of cis-1,3-cyclohexanediol was 75%.

The reaction solution was filtered, and the residue was washed 3 times with 5 ml of tetrahydrofuran. The wash liquids and filtrates were combined, and 86 ml of tetrahydrofuran was evaporated under a reduced pressure. The residue was mixed with 100 ml of toluene and stirred at room temperature for 15 minutes, and then 88 ml of toluene and water as a mixture was evaporated by azeotropic dehydration under a reduced pressure. The thus obtained oily matter was dissolved in 76 ml of a 18:7 mixed solvent of ethyl acetate and toluene, and the solution was cooled from room temperature to -31.5°C spending 2.5 hours, thereby obtaining 25.7 g of cis-1,3-cyclohexanediol as white crystals (cis-form selectivity of the crystals was 94%, and crystal yield was 73.8%).

Example 2

A reaction vessel was charged with 511.8 mg (13.5 mmol) of sodium borohydride, 8 ml of tetrahydrofuran, 296 mg (2.3 mmol) of normal nonane and 655.4 mg (6.88 mmol) of magnesium chloride, and the contents were stirred at room temperature for 45 minutes. To this was added 10 ml of tetrahydrofuran solution containing 1.11 g (9.9 mmol) of 1,3-cyclohexanedione, spending 30 minutes. Thereafter, the reaction and after-treatment were carried out in accordance with Example 1. 1,3-Cyclohexanediol was formed in the reaction solution with a yield of 73.8%. The by-products were cyclohexanol (0.4%) and 2-cyclohexen-1-ol (0.9%). Also, conversion ratio of 1,3-cyclohexanedione was 96.5%, and selectivity of cis-1,3-cyclohexanediol was 60%.

Example 3

A reaction vessel was charged with 510 mg (13.5 mmol) of sodium borohydride, 8 ml of tetrahydrofuran, 209 ml (1.63 mmol) of normal nonane and 650 mg (6.8 mmol) of magnesium chloride, and the contents were stirred at room temperature for 24 hours. To this was added 9.8 ml of tetrahydrofuran solution containing 1.11 g (9.9 mmol) of 1,3-cyclohexanedione, spending 30 minutes. Thereafter, the reaction and after-treatment were carried out in accordance with Example 1. 1,3-Cyclohexanediol was formed in the reaction solution with a yield of 97.5%. The by-products were cyclohexanol (0.2%) and 2-cyclohexen-1-ol (1.2%). Also, conversion ratio of 1,3-cyclohexanedione was 99.9%, and selectivity of cis-1,3-cyclohexanediol was 75%.

Example 4

A reaction vessel was charged with 462.5 mg (12.2 mmol) of sodium borohydride, 8 ml of tetrahydrofuran, 237.5 mg (1.85 mmol) of normal nonane and 737.5 mg (6.13 mmol) of magnesium sulfate, and the contents were stirred at room temperature for 45 minutes. Then, 9.8 ml of tetrahydrofuran solution containing 1.11 g (9.9 mmol) of 1,3-cyclohexanedione was added spending 30 minutes. Thereafter, the reaction and after-treatment were carried out in accordance with Example 1. 1,3-Cyclohexanediol was formed in the reaction solution with a yield of 65.5%. The by-products were cyclohexanol (14.9%) and 2-cyclohexen-1-ol (9.5%). Also, conversion ratio of 1,3-cyclohexanedione was 98.0%, and selectivity of cis-1,3-cyclohexanediol was 48%.

Example 5

A reaction vessel was charged with 514 mg (13.6 mmol) of sodium borohydride, 5 ml of tetrahydrofuran, 208.8 mg of normal nonane and 906 mg (6.8 mmol) of aluminum chloride, and the contents were stirred at room temperature for 45 minutes. To this was added dropwise 10 ml of tetrahydrofuran solution containing 1.01 g (9 mmol) of 1,3-cyclohexanedione, spending 30 minutes. After completion of the dropwise addition, the mixture was heated to 60°C and allowed to undergo the reaction for 25 hours by keeping this temperature. After spontaneous cooling of the reaction solution and subsequent cooling to about 0°C, 2.7 ml of water was added thereto. Thereafter, this was returned to room temperature and stirred for 1 hour. 1,3-Cyclohexanediol was formed in the reaction solution with a yield of 70.0%. The by-products were cyclohexanol (10.7%) and 2-cyclohexen-1-ol (4.4%). Also, conversion ratio of 1,3-cyclohexanedione was 98.1%, and selectivity of cis-1,3-cyclohexanediol was 32%.

Example 6

A reaction vessel was charged with 510 mg (13.5 mmol) of sodium borohydride, 8 ml of tetrahydrofuran, 237.5 mg (1.85 mmol) of normal nonane and 752.8 mg (6.8 mmol) of calcium chloride, and the contents were stirred at room temperature for 24 hours. To this was added 9.8 ml of tetrahydrofuran solution containing 1.11 g (9.9 mmol) of 1,3-cyclohexanedione, spending 30 minutes. Thereafter, the reaction and after-treatment were carried out in accordance with Example 1. 1,3-Cyclohexanediol was formed in the reaction solution with a yield of 73.2%. The by-products were cyclohexanol (3.5%) and 2-cyclohexen-1-ol (2.2%). Also, conversion ratio of 1,3-cyclohexanedione was 83.3%, and selectivity of cis-1,3-cyclohexanediol was 31%.

Example 7

A reaction vessel was charged with 510 mg (13.5 mmol) of sodium borohydride, 8 ml of tetrahydrofuran, 237.5 mg (1.85 mmol) of normal nonane and 288.7 mg (6.8 mmol) of lithium chloride, and the contents were stirred at room temperature for 24 hours. To this was then added 9.8 ml of tetrahydrofuran solution containing 1.11 g (9.9 mmol) of 1,3-cyclohexanedione, spending 30 minutes. Thereafter, the reaction and after-treatment were carried out in accordance with Example 1. 1,3-Cyclohexanediol was formed in the reaction solution with a yield of 98.6. Also, conversion ratio of 1,3-cyclohexanedione was 99.7%, and selectivity of cis-1,3-cyclohexanediol was 71%.

Example 8

A reaction vessel was charged with 510 mg (13.5 mmol) of sodium borohydride, 8 ml of tetrahydrofuran, 237.5 mg (1.85 mmol) of normal nonane and 918.2 mg (6.8 mmol) of zinc chloride, and the contents were stirred at room temperature for 24 hours. To this was added dropwise 9.8 ml of tetrahydrofuran solution containing 1.11 g (9.9 mmol) of 1,3-cyclohexanedione, spending 30 minutes. After completion of the dropwise addition, the mixture was heated to 60°C and allowed to undergo the reaction for 8 hours by keeping this temperature. After spontaneous cooling of the

reaction solution and subsequent cooling to about 0°C, 2.7 ml of water was added thereto. Thereafter, this was returned to room temperature and stirred for 1 hour. 1,3-Cyclohexanediol was formed in the reaction solution with a yield of 66.6%. The by-products were cyclohexanol (12.5%) and 2-cyclohexen-1-ol (20.9%). Also, conversion ratio of 1,3-cyclohexanedione was 100%, and selectivity of cis-1,3-cyclohexanediol was 42%.

Example 9

A four neck flask equipped with a condenser, a thermometer and a mechanical stirrer was charged with 237.8 mg (1.85 mmol) of normal nonane and 1.01 g (9 mmol) of 1,3-cyclohexanedione dissolved in 30 ml of tetrahydrofuran. Next, to this was added 679.3 mg (18 mmol) of sodium borohydride. The mixture was heated to 60°C and stirred for 27 hours. After spontaneous cooling of the reaction solution, 4.5 ml of 1 N hydrochloric acid was added thereto. 1,3-Cyclohexanediol was formed in the reaction solution with a yield of 75.3%. The by-products were cyclohexanol (9.1%) and 2-cyclohexen-1-ol (5.6%). Also, conversion ratio of 1,3-cyclohexanedione was 90.0%, and selectivity of trans-1,3-cyclohexanediol was 89%.

Example 10

A four neck flask equipped with a condenser, a thermometer and a mechanical stirrer was charged with 115.5 mg (0.9 mmol) of normal nonane and 1.01 g (9 mmol) of 1,3-cyclohexanedione dissolved in 30 ml of tetrahydrofuran. Next, to this was added 170.1 mg (4.5 mmol) of sodium borohydride. The mixture was-heated to 60°C and stirred for 27 hours. After spontaneous cooling of the reaction solution, 4.5 ml of 1 N hydrochloric acid was added thereto. 1,3-Cyclohexanediol was formed in the reaction solution with a yield of 70.6%. The by-products were cyclohexanol (3.3%) and 2-cyclohexen-1-ol (1.8%). Also, conversion ratio of 1,3-cyclohexanedione was 75.7%, and selectivity of trans-1,3-cyclohexanediol was 96%.

Comparative Example 1

The reaction was carried out using a general aluminum system reducing agent, lithium aluminum hydride, instead of a boron hydride compound.

That is, a three neck flask equipped with a thermometer and a three-way cock was charged at room temperature with 0.68 g (18.0 mmol) of lithium aluminum hydride, 20 ml of tetrahydrofuran and 0.24 g (1.9 mmol) of normal nonane as the internal standard for analysis. A 1.01 g (9.0 mmol) portion of 1,3-cyclohexanedione dissolved in 10 ml of THF was added to this solution and stirred at room temperature for 21 hours. This reaction solution was cooled to 0°C, 3.6 ml of 0.05 N hydrochloric acid aqueous solution was added dropwise thereto, and then the temperature was increased to room temperature. When the thus obtained solution was analyzed by a gas chromatography and a liquid chromatography, conversion ratio of 1,3-cyclohexanedione was 99.4%, and the main product was 2-cyclohexen-1-ol (yield 27.1%). Other product was cyclohexanol (15.0%), and the 1,3-cyclohexanediol of interest was formed only in a low yield of 10.2%.

Comparative Example 2

The reaction was carried out using a general aluminum system reducing agent, sodium bis(2-methoxyethoxy) aluminum hydride, instead of a boron hydride compound.

That is, 1.02 g (9.1 mmol) of 1,3-cyclohexanedione and 0.23 g (1.8 mmol) of normal nonane as the internal standard for analysis were suspended in 20 ml of toluene at room temperature in a three neck flask equipped with a thermometer and a three-way cock. When 5.4 ml (18.0 mmol) of a 65% toluene solution of sodium bis(2-methoxyethoxy) aluminum hydride was added thereto at room temperature, it became a uniform solution, and the solution was stirred at the same temperature for 17.5 hours. This reaction solution was cooled to 0°C, 6 ml of methanol was added dropwise thereto, and then the temperature was returned to room temperature. When the thus obtained solution was analyzed by a gas chromatography and a liquid chromatography, conversion ratio of 1,3-cyclohexanedione was 77.2%, the main product was 2-cyclohexen-1-ol (yield 76.7%) and other product was cyclohexanol (2.2%), and formation of the 1,3-cyclohexanediol of interest could not be observed.

According to the invention, 1,3-cyclohexanediol compounds, particularly cis-1,3-cyclohexanediol compounds, can be produced industrially advantageously and efficiently by a relatively simple process with a low cost.

While the present invention has been described in detail and with reference to specific embodiments thereof, it will be apparent to one skilled in the art that various changes and modifications can be made therein without departing from the scope thereof.

This application is based on Japanese patent application No. 2002-028217 filed February 5, 2002, the entire

contents thereof being hereby incorporated by reference.

**Claims**

1. A method for producing a 1,3-cyclohexanediol compound represented by the following general formula (2)

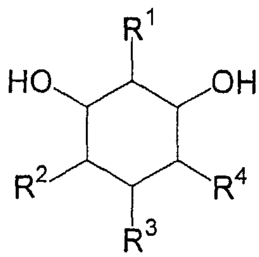

(2)

(wherein $R^1$, $R^2$, $R^3$ and $R^4$ each independently represents hydrogen atom, a halogen atom, an alkoxycarbonyl group, an alkyl group which may have one or more substituent(s), an alkoxy group which may have one or more substituent(s) or phenyl group-which may have one or more substituent(s)) which comprises reducing a 1,3-cyclohexanedione compound represented by the following general formula (1)

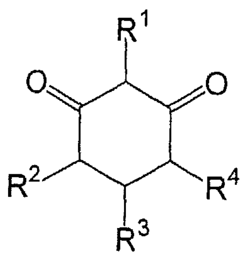

(1)

(wherein $R^1$, $R^2$, $R^3$ and $R^4$ are as defined in the formula (2)) with a boron hydride compound.

2. The method according to claim 1, wherein each of $R^1$, $R^2$, $R^3$ and $R^4$ is hydrogen atom.

3. The method according to claim 1 or 2, wherein the reduction is carried out in the presence of one or two or more metal compounds selected from the group I, group II, group XII or group XIII elements of the periodic table, in addition to the boron hydride compound.

4. The production method according to claim 3, wherein the metal compound selected from the group I, group II, group XII or group XIII elements of the periodic table, to be used in addition to the boron hydride compound, is a lithium compound or a magnesium compound.

European Patent Office

# EUROPEAN SEARCH REPORT

Application Number
EP 03 00 2527

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | DATABASE CAPLUS [Online] CHEMICAL ABSTRACTS SERVICE, COLUMBUS, OHIO, US; 1968 PONOMAREV A A & POPOVA L V: "Cis- and trans-1,3-cyclohexanediols" retrieved from STN Database accession no. 1968:451707 XP002241288 * abstract * & SU 201 364 A (..) 8 September 1968 (1968-09-08) --- | 1-4 | C07C29/143 C07C35/14 |
| Y | HICKMOTT P W ET AL.: "Enamine Chemistry. Part 29. Synthesis of Adamantane Derivatives from alpha-beta-Unsaturated Acid Chlorides and 4,4-Disubstituted Cyclohexanone Enamines. Multiple [3,3] Sigmatropic Rearrangement Transition State Stereochemistry. X-Ray analysis." JOURNAL OF THE CHEMICAL SOCIETY PERKIN TRANSACTIONS 1, 1985, pages 2559-2572, XP009010560 * page 2568, column 2, line 29 - line 52 * --- | 1-4 | TECHNICAL FIELDS SEARCHED (Int.Cl.7) C07C |
| Y | BERG U ET AL.: "Stereochemistry of the Reduction of Bicyclo[3.3.1]nonane-2,9-dione by Complex Hydrides" JOURNAL OF THE CHEMICAL SOCIETY PERKIN TRANSACTIONS 2, 1995, pages 97-102, XP009010561 * page 98; figure 1; table 1 * --- | 1-4 | |
| A | US 3 647 890 A (HUTCHINS JONATHAN E C ET AL) 7 March 1972 (1972-03-07) * example 1 * --- -/-- | 1-4 | |

The present search report has been drawn up for all claims

| Place of search | Date of completion of the search | Examiner |
|---|---|---|
| MUNICH | 16 May 2003 | Janus, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 00 2527

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DE 28 11 918 A (BAYER AG) 27 September 1979 (1979-09-27) * example 1 * --- | 1-4 | |
| A | MARSHALL J A & SCANTO C J V: "Synthesis of Substituted cis-Cyclodecenes from Cyclooctanone" JOURNAL OF ORGANIC CHEMISTRY, vol. 30, September 1965 (1965-09), pages 3019-3023, XP002241287 * example 5A * ----- | 1-4 | |

TECHNICAL FIELDS SEARCHED (Int.Cl.7)

The present search report has been drawn up for all claims

| Place of search | Date of completion of the search | Examiner |
|---|---|---|
| MUNICH | 16 May 2003 | Janus, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 03 00 2527

*This annex lists the patent family members relating to the patent documents cited in the above-mentioned European search report.*
The members are as contained in the European Patent Office EDP file on
The European Patent Office is in no way liable for these particulars which are merely given for the purpose of information.

16-05-2003

| Patent document cited in search report | | Publication date | Patent family member(s) | | Publication date |
|---|---|---|---|---|---|
| SU 201364 | A | | NONE | | |
| US 3647890 | A | 07-03-1972 | NONE | | |
| DE 2811918 | A | 27-09-1979 | DE | 2811918 A1 | 27-09-1979 |

EPO FORM P0459

For more details about this annex : see Official Journal of the European Patent Office, No. 12/82